# EUROPEAN PATENT APPLICATION

(11) **EP 4 834 314 A1**
(43) Date of publication of application: **04.11.2026**
(21) Application number: 24912449.6
(22) Date of filing: 13.12.2024
(51) Int. Cl.: A61K 8/29, A61K 8/06, A61K 8/81, A61K 8/86, A61Q 1/00, A61Q 19/00

(54) **OIL-IN-WATER-TYPE EMULSION COMPOSITION**

(30) Priority: 28.12.2023 JP 2023223525
(71) Applicant: Shiseido Company, Ltd., Chuo-ku Tokyo 104-0061 (JP)
(72) Inventor: SUN, Yudi, Tokyo 104-0061 (JP); KATORI, Takahiro, Tokyo 104-0061 (JP)
(74) Representative: Ter Meer und Partner mbB
(86) International application number: PCT/JP2024/044232
(87) International publication number: WO 2025/142560

(57) **Abstract**

An oil-in-water emulsion composition includes (A) an inorganic powder treated with a monoalkyl phosphate; (B) a polyoxyethylene hydrogenated castor oil; and (C) an acrylamide-based thickener.

## Description

### TECHNICAL FIELD

The present invention relates to an oil-in-water emulsion composition.

### BACKGROUND ART

In recent years, there has been a trend in countries that materials having high safety for the human body and conforming to the EU standards are required to be used as various materials included in cosmetics.
Among the materials included in cosmetics, surface-treated metal oxides are often used as an ultraviolet scattering agent in cosmetics, such as sunscreens, foundations, and the like. For example, Patent Document 1 discloses zinc oxide particles surface-treated with octyltriethoxysilane, which have ultraviolet shielding properties, excellent color and feelings during use, and applicability to cosmetics.

### RELATED ART DOCUMENTS

### PATENT DOCUMENTS

Patent Document 1: Japanese Patent Application Publication No. 2023-162678

### SUMMARY OF THE INVENTION

### PROBLEM TO BE SOLVED BY THE INVENTION

However, there is a need for use of a surface treatment agent having safety higher than that of octyltriethoxysilane used as the surface treatment agent for the zinc oxide particles in Patent Document 1.

For the treatment, however, it is very difficult to replace a surface treatment agent used for a conventional ultraviolet scattering agent, such as octyltriethoxysilane, with a surface treatment agent considered to have higher safety. Specifically, the surface treatment of zinc oxide, titanium oxide, or the like forming the cores of the ultraviolet scattering agent becomes insufficient, and the emulsion stability tends to deteriorate when the resulting product is compounded in cosmetics or the like.

It is an object of an embodiment of the present invention to provide an oil-in-water emulsion composition having high safety and good emulsion stability.

### MEANS FOR SOLVING THE PROBLEM

In order to solve the above problem, the embodiment of the present invention provides an oil-in-water emulsion composition including:
(A) an inorganic powder treated with a monoalkyl phosphate;
(B) a polyoxyethylene hydrogenated castor oil; and
(C) an acrylamide-based thickener.

### ADVANTAGEOUS EFFECTS OF THE INVENTION

According to the embodiment of the present invention, it is possible to provide an oil-in-water emulsion composition having high safety and good emulsion stability.

### EMBODIMENTS FOR CARRYING OUT THE INVENTION

Embodiments for carrying out the present invention will be described below in detail.

### (Oil-in-water emulsion composition)

The oil-in-water emulsion composition of the present invention is an oil-in-water emulsion composition including:
(A) an inorganic powder treated with a monoalkyl phosphate;
(B) a polyoxyethylene hydrogenated castor oil; and
(C) an acrylamide-based thickener.

According to the present invention, it is possible to provide an oil-in-water emulsion composition having high safety and good emulsion stability.

### <(A) Inorganic powder treated with monoalkyl phosphate>

The oil-in-water emulsion composition of the present invention includes, as component (A), an inorganic powder treated with a monoalkyl phosphate. The inorganic powder treated with the monoalkyl phosphate is an inorganic powder obtained by hydrophobizing fine particles, to serve as the cores of the inorganic powder, with the monoalkyl phosphate. The inorganic powder treated with the monoalkyl phosphate functions as an ultraviolet scattering agent in the oil-in-water emulsion composition of the present invention.

The monoalkyl phosphate is a surface treatment agent having higher safety for the human body than the surface treatment agent used in conventional ultraviolet scattering agents, such as octyltriethoxysilane.

The inorganic powder treated with the monoalkyl phosphate is stable in oil, and contributes to the emulsion stability of the oil-in-water emulsion composition of the present invention.

No particular limitation is imposed on the amount of (A) the inorganic powder treated with the monoalkyl phosphate in the present invention, and it is preferably 1% by mass or more and 10% by mass or less, more preferably 2% by mass or more and 7% by mass or less, and yet more preferably 3% by mass or more and 5% by mass or less. When it is 1% by mass or more, the effect as an ultraviolet scattering agent can be sufficiently ensured. When it is 10% by mass or less, the emulsion stability is readily maintained.

(A) the inorganic powder treated with the monoalkyl phosphate functioning as an ultraviolet scattering agent may be in a powdery form. In this case, no particular limitation is imposed on the average primary particle diameter, and it may be, for example, 5 nm to 200 nm, 8 nm to 100 nm, 10 nm to 50 nm, or 12 nm to 30 nm. The "average primary particle diameter" may be determined, for example, as a projected area equivalent circle diameter of the primary particles in an SEM image.

### <<Inorganic powder>>

Examples of the inorganic powder treated with the monoalkyl phosphate usable for the oil-in-water emulsion composition of the present invention include those obtained by performing a hydrophobizing surface treatment with the monoalkyl phosphate on a metal oxide powder in the form of fine particles, such as titanium oxide, zinc oxide, iron oxide, cerium oxide, tungsten oxide, or the like.

Examples of the monoalkyl phosphate include cetyl phosphate, ethyl phosphate, octyl phosphate, stearyl phosphate, dodecyl phosphate, and the like. It is preferable to include cetyl phosphate.

The inorganic powder preferably includes a silica coating. Specifically, examples thereof include those obtained by performing a hydrophobizing surface treatment with the monoalkyl phosphate on a silica coating-including surface of a metal oxide powder in the form of fine particles, such as titanium oxide, zinc oxide, iron oxide, cerium oxide, tungsten oxide, or the like. When the inorganic powder includes a silica coating, the silica coating is preferably one or more layers, and more preferably two or more layers, over the entire surfaces of the metal oxide powder in the form of fine particles.

When the inorganic powder includes the silica coating, the surfaces of the fine particles of the metal oxide are covered by silica. This suppresses the catalytic activity of the metal, thereby providing good stability during use in combination with an absorber. Therefore, it is possible to suppress discoloration and improve texture during use. Also, when the silica coating is two or more layers, such effects can be further improved.

### <(B) Polyoxyethylene hydrogenated castor oil>

The oil-in-water emulsion composition includes a polyoxyethylene hydrogenated castor oil as component (B). The polyoxyethylene hydrogenated castor oil is a nonionic surfactant, and contributes to the emulsion stability of the oil-in-water emulsion composition of the present invention.

As the average number of moles of ethylene oxide added increases, the hydrophilicity of the polyoxyethylene hydrogenated castor oil increases, i.e., the emulsion stability improves. Thus, the average number of moles of ethylene oxide added in the polyoxyethylene hydrogenated castor oil in the present invention is preferably 50 mol or more and 100 mol or less. When the average number of moles of ethylene oxide added in the polyoxyethylene hydrogenated castor oil is 50 mol or more, the sufficient hydrophilicity is readily ensured, i.e., the emulsion stability is readily improved. When the average number of moles of ethylene oxide added in the polyoxyethylene hydrogenated castor oil is 100 mol or less, stickiness is reduced, resulting in a good feeling during use.

The amount of the polyoxyethylene hydrogenated castor oil in the oil-in-water emulsion composition of the present invention is preferably 0.1% by mass or more and 10% by mass or less, more preferably 0.5% by mass or more and 5% by mass or less, and yet more preferably 1% by mass or more and 3% by mass or less. When the amount of the polyoxyethylene hydrogenated castor oil is 0.1% by mass or more, the emulsion stability can be sufficiently improved. When it is 10% by mass or less, stickiness derived from the surfactant is reduced, resulting in a good feeling during use.

Examples of the polyoxyethylene hydrogenated castor oil suitable for the oil-in-water emulsion composition of the present invention include, for example, a PEG-60 hydrogenated castor oil and a PEG-100 hydrogenated castor oil.

### <(C) Acrylamide-based thickener>

The oil-in-water emulsion composition of the present invention includes an acrylamide-based thickener as component (C). The acrylamide-based thickener has a thickening function to thicken an aqueous phase by swelling of the polymer, and contributes to an increase in the emulsion stability of the oil-in-water emulsion composition of the present invention.

Examples of the acrylamide-based thickener include, for example, a (dimethylacrylamide/Na acryloyldimethyl taurate) crosspolymer, a (Na acrylate/Na acryloyldimethyl taurate) copolymer, an (ammonium acryloyldimethyl taurate/beheneth-25 methacrylate) crosspolymer, an (ammonium acryloyldimethyl taurate/VP) copolymer, and a (hydroxyethyl acrylate/Na acryloyldimethyl taurate) copolymer.

The amount of the acrylamide-based thickener in the oil-in-water emulsion composition of the present invention is preferably 0.01% by mass or more and 2% by mass or less, more preferably 0.05% by mass or more and 1% by mass or less, and yet more preferably 0.1% by mass or more and 0.5% by mass or less. When the amount of the acrylamide-based thickener is 0.01% by mass or more, a sufficient viscosity can be readily ensured. When it is 2% by mass or less, cosmetics and the like have an appropriate viscosity, resulting in a good feeling during use because of a less filmy feel.

A ratio between the amount of (B) the polyoxyethylene hydrogenated castor oil : the amount of (C) the acrylamide-based thickener is preferably 100:1 to 2:1 on a mass basis, more preferably 60:1 to 3:1, and yet more preferably 30:1 to 5:1. When the ratio between the amount of (B) the polyoxyethylene hydrogenated castor oil : the amount of (C) the acrylamide-based thickener is 100:1 to 2:1, both stability and feeling during use can be achieved.

### <Other ingredients>

The oil-in-water emulsion composition of the present invention can be produced by a routine method in accordance with the intended dosage form by appropriately compounding ingredients other than the above, if necessary, such as powder ingredients, liquid oils and fats, solid oils and fats, waxes, hydrocarbon oils, higher fatty acids, higher alcohols, synthetic ester oils, silicone oils, silicones, anionic surfactants, cationic surfactants, amphoteric surfactants, nonionic surfactants, active agents other than surfactants, water-soluble polymers, thickeners, ultraviolet absorbers, humectants, metal ion sequestrants, lower alcohols, polyhydric alcohols, sugars, amino acids, organic amines, polymer emulsions, neutralizing agents, pH adjusters, vitamins, antioxidants, antioxidant aids, stabilizers, perfumes, water, and the like. Specific compoundable ingredients are listed below.

Examples of the powder ingredients include, for example: inorganic powders (e.g., talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, metal tungstate, magnesium, silica, zeolite, barium sulfate, calcined calcium sulfate (calcined gypsum), calcium phosphate, fluorapatite, hydroxyapatite, ceramic powder, metal soap (e.g., zinc myristate, calcium palmitate, and aluminum stearate), and boron nitride); organic powders (e.g., polyamide resin powder (nylon powder), polyethylene powder, polymethyl methacrylate powder, polystyrene powder, copolymer resin powder of styrene and acrylic acid, benzoguanamine resin powder, polytetrafluoroethylene powder, and cellulose powder); inorganic white pigments (e.g., titanium dioxide and zinc oxide); inorganic red pigments (e.g., iron oxide (red iron oxide) and iron titanate); inorganic brown pigments (e.g., γ-iron oxide); inorganic yellow pigments (e.g., iron yellow oxide and yellow ochre); inorganic black pigments (e.g., black iron oxide and lower titanium oxide); inorganic violet pigments (e.g., manganese violet and cobalt violet); inorganic green pigments (e.g., chromium oxide, chromium hydroxide, and cobalt titanate); inorganic blue pigments (e.g., ultramarine blue and Prussian blue); pearl pigments (e.g., titanium oxide-coated mica, titanium oxide-coated bismuth oxychloride, titanium oxide-coated talc, colored titanium oxide-coated mica, bismuth oxychloride, and fish scale foils); metal powder pigments (e.g., aluminum powder and copper powder); organic pigments, such as zirconium, barium, aluminum lake, and the like (e.g., organic pigments, such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404; and Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1); and natural pigments (e.g., chlorophyll and β-carotene).

Examples of the liquid fats and oils include, for example: avocado oil, camellia oil, turtle oil, macadamia nut oil, corn oil, mink oil, olive oil, rapeseed oil, egg yolk oil, sesame oil, apricot kernel oil, wheat germ oil, camellia kissi seed oil, castor oil, linseed oil, safflower oil, cottonseed oil, perilla oil, soybean oil, peanut oil, camellia sinensis seed oil, kaya nut oil, rice bran oil, Chinese tung oil, Japanese tung oil, jojoba oil, germ oil, and triglycerin.

Examples of the solid fats and oils include, for example, cacao butter, coconut oil, horse oil, hydrogenated coconut oil, palm oil, beef tallow, mutton tallow, hydrogenated beef tallow, palm kernel oil, lard, beef bone fat, Japan wax kernel oil, hydrogenated oil, beef leg fat, Japan wax, and hydrogenated castor oil.

Examples of the waxes include, for example, beeswax, candelilla wax, cotton wax, carnauba wax, bayberry wax, insect wax, whale wax, montane wax, bran wax, lanolin, kapok wax, lanolin acetate, liquid lanolin, sugar cane wax, isopropyl lanolate, hexyl laurate, reduced lanolin, jojoba wax, hard lanolin, shellac wax, POE lanolin alcohol ether, POE lanolin alcohol acetate, POE cholesterol ether, polyethylene glycol lanolate, and POE hydrogenated lanolin alcohol ether.

Examples of the hydrocarbon oils include, for example, liquid paraffin, ozokerite, squalane, pristane, paraffin, ceresin, squalene, petrolatum, and microcrystalline wax.
Examples of the higher fatty acids include, for example, lauric acid, myristic acid, palmitic acid, stearic acid, behenic acid, oleic acid, undecylenic acid, tall acid, isostearic acid, linoleic acid, linolenic acid, eicosapentaenoic acid (EPA), and docosahexaenoic acid (DHA).

Examples of the higher alcohols include, for example: straight-chain alcohols (e.g., lauryl alcohol, cetyl alcohol, stearyl alcohol, behenyl alcohol, myristyl alcohol, oleyl alcohol, and cetostearyl alcohol); and branched-chain alcohols (e.g., monostearyl glyceryl ether (batyl alcohol), 2-decyltetradecinol, lanolin alcohol, cholesterol, phytosterol, hexyldodecanol, isostearyl alcohol, and octyldodecanol).

Examples of the synthetic ester oils include, for example: isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glyceryl di-2-heptylundecanoate, trimethylolpropane tri-2-ethylhexanoate, trimethylolpropane triisostearate, pentaerythritol tetra-2-ethylhexanoate, glyceryl tri-2-ethylhexanoate, glyceryl trioctanoate, glyceryl triisopalmitate, trimethylolpropane triisostearate, cetyl 2-ethylhexanoate, 2-ethylhexyl palmitate, glyceryl trimyristate, glyceryl tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate.

Examples of the silicone oils include, for example: chain polysiloxanes (e.g., dimethylpolysiloxane, methylphenylpolysiloxane, and diphenylpolysiloxane); cyclic polysiloxanes (e.g., octamethylcyclotetrasiloxane, decamethylcyclopentasiloxane, and dodecamethylcyclohexasiloxane); silicone resins forming three-dimensional networks; silicone rubber; and various modified polysiloxanes (e.g., amino-modified polysiloxane, polyether-modified polysiloxanes, alkyl-modified polysiloxanes, and fluorine-modified polysiloxanes).

Examples of the silicones include, for example: alkyl and alkoxy silicone waxes, such as stearyl-modified dimethicone, behenyl-modified dimethicone, and the like; silicone resins forming three-dimensional networks; and silicone rubber.

Examples of the anionic surfactants include, for example: fatty acid soaps (e.g., sodium laurate and sodium palmitate); higher alkyl sulfate salts (e.g., sodium lauryl sulfate and potassium lauryl sulfate); alkyl ether sulfate salts (e.g., POE-lauryl sulfate triethanolamine and POE-sodium lauryl sulfate); N-acylsarcosic acids (e.g., sodium lauroylsarcosinate); higher fatty acid amide sulfonate salts (e.g., sodium N-myristoyl-N-methyltaurate, sodium coconut oil fatty acid methyltauride, and sodium laurylmethyltauride); phosphate salts (POE-sodium oleyl ether phosphate and POE-stearyl ether phosphate); sulfosuccinate salts (e.g., sodium di-2-ethylhexyl sulfosuccinate, sodium monolauroyl monoethanolamide polyoxyethylene sulfosuccinate, and sodium lauryl polypropylene glycol sulfosuccinate); alkylbenzene sulfonate salts (e.g., sodium linear dodecylbenzenesulfonate, triethanolamine linear dodecylbenzenesulfonate, and linear dodecylbenzenesulfonic acid); higher fatty acid ester sulfate salts (e.g., sodium hydrogenated glyceryl cocoate sulfate); N-acylglutamate salts (e.g., monosodium N-lauroylglutamate, disodium N-stearoylglutamate, and monosodium N-myristoyl-L-glutamate); sulfated oils (e.g., rote oil); POE-alkyl ether carboxylates; POE-alkyl allyl ether carboxylate salts; α-olefin sulfonate salts; higher fatty acid ester sulfonate salts; secondary alcohol sulfate salts; higher fatty acid alkylolamide sulfate salts; sodium lauroyl monoethanolamide succinate; N-palmitoylaspartic acid ditriethanolamine; and sodium caseinate.

Examples of the cationic surfactants include, for example: alkyltrimethylammonium salts (e.g., stearyltrimethylammonium chloride and lauryltrimethylammonium chloride); alkylpyridinium salts (e.g., cetylpyridinium chloride); distearyldimethylammonium chloride and dialkyldimethylammonium salts; poly(N,N'-dimethyl-3,5-methylenepiperidinium) chloride; alkyl quaternary ammonium salts; alkyldimethylbenzylammonium salts; alkylisoquinolinium salts; dialkylmolyphonium salts; POE-alkylamines; alkylamine salts; polyamine fatty acid derivatives; amyl alcohol fatty acid derivatives; benzalkonium chloride; and benzethonium chloride.

Examples of the amphoteric surfactants include, for example: imidazoline-based amphoteric surfactants (e.g., 2-undecyl-N,N,N-(hydroxyethylcarboxymethyl)-2-imidazoline sodium and 2-cocoyl-2-imidazolinium hydroxide-1-carboxyethyloxy 2 sodium salt); betaine-based surfactants (e.g., 2-heptadecyl-N-carboxymethyl-N-hydroxyethylimidazolinium betaine, lauryldimethylaminoacetic acid betaine, alkyl betaine, amide betaine, and sulfobetaine).

Examples of lipophilic nonionic surfactants include, for example: sorbitan fatty acid esters (e.g., sorbitan monooleate, sorbitan monoisostearate, sorbitan monolaurate, sorbitan monopalmitate, sorbitan monostearate, sorbitan sesquioleate, sorbitan trioleate, penta-2-ethylhexylic acid diglycerol sorbitan, and tetra-2-ethylhexylic acid diglycerol sorbitan); glyceryl/polyglyceryl fatty acid esters (e.g., mono-cottonseed oil fatty acid glycerin, mono-erucic acid glycerin, sesquioleic acid glycerin, monostearic acid glycerin, α,α'-oleic acid pyroglutamic acid glycerin, and monostearic acid glycerin malic acid); propylene glycol fatty acid esters (e.g., propylene glycol monostearate); hydrogenated castor oil derivatives; and glyceryl alkyl ethers.

Examples of hydrophilic nonionic surfactants include, for example: POE-sorbitan fatty acid esters (e.g., POE-sorbitan monooleate, POE-sorbitan monostearate, POE-sorbitan monooleate, and POE-sorbitan tetraoleate); POE-sorbit fatty acid esters (e.g., POE-sorbit monolaurate, POE-sorbit monooleate, POE-sorbit pentaoleate, and POE-sorbit monostearate); POE-glycerin fatty acid esters (e.g., POE-monooleates, such as POE-glyceryl monostearate, POE-glyceryl monoisostearate, and POE-glyceryl triisostearate); POE-fatty acid esters (e.g., POE-distearate, POE-monodioleate, and ethylene glycol distearate); POE-alkyl ethers (e.g., POE-lauryl ether, POE-oleyl ether, POE-stearyl ether, POE-behenyl ether, POE-2-octyldodecyl ether, and POE-cholestanol ether); PLURONIC (registered trademark)-type surfactants (e.g., PLURONIC (registered trademark)); POE-POP-alkyl ethers (e.g., POE-POP-cetyl ether, POE-POP-2-decyltetradecyl ether, POE-POP-monobutyl ether, POE-POP-hydrogenated lanolin, and POE-POP-glycerin ether); tetra-POE-tetra-POP-ethylenediamine condensates (e.g., Tetronic); POE-castor oil-hydrogenated castor oil derivatives (e.g., POE-castor oil, POE-hydrogenated castor oil, POE-hydrogenated castor oil monoisostearate, POE-hydrogenated castor oil triisostearate, POE-hydrogenated castor oil monopyroglutamic acid monoisostearic acid diester, and POE-hydrogenated castor oil maleic acid); POE-beeswax-lanolin derivatives (e.g., POE-sorbit beeswax); alkanolamides (e.g., coconut oil fatty acid diethanolamide, lauric acid monoethanolamide, and fatty acid isopropanolamide); POE-propylene glycol fatty acid esters; POE-alkylamines; POE-fatty acid amides; sucrose fatty acid esters; alkylethoxydimethylamine oxides; and trioleyl phosphoric acid.

Examples of the active agents other than surfactants include, for example, active agents for emulsifying by use of fine particles, and specifically include, for example, active agents for Pickering emulsification. Examples of the active agents for Pickering emulsification include, for example, a (acrylamide/DMAPA acrylic acid/methoxy PEG methacrylic acid) copolymer and hydrophobic silica. Since the (acrylamide/DMAPA acrylic acid/methoxy PEG methacrylic acid) copolymer has water resistance, it can be successfully used when water resistance is required for the oil-in-water emulsion composition.

Examples of natural water-soluble polymers include, for example: plant polymers (e.g., gum Arabic, gum tragacanth, galactan, guar gum, carob gum, karaya gum, carrageenan, pectin, agar, quince seed (marmelo), algal colloid (algal extract), starch (rice, corn, potatoes, and wheat), and glycyrrhizic acid); and microorganism-derived polymers (e.g., xanthan gum, dextran, succinoglycan, and pullulan).

Examples of semisynthetic water-soluble polymers include, for example: starch-based polymers (e.g., carboxymethyl starch and methyl hydroxypropyl starch); cellulose-based polymers (e.g., methyl cellulose, ethyl cellulose, methyl hydroxypropyl cellulose, hydroxyethyl cellulose, sodium cellulose sulfate, hydroxypropyl cellulose, carboxymethyl cellulose, sodium carboxymethyl cellulose, crystalline cellulose, and cellulose powder); alginate-based polymers (e.g., sodium alginate and propylene glycol alginate).

Examples of synthetic water-soluble polymers include, for example: vinyl-based polymers (e.g., polyvinyl alcohol, polyvinyl methyl ether, polyvinyl pyrrolidone, and carboxyvinyl polymer); polyoxyethylene-based polymers (e.g., copolymers between polyoxyethylenes of polyethylene glycols 20,000, 40,000, and 60,000, and polyoxypropylenes); acrylic polymers (e.g., sodium polyacrylate, polyethyl acrylate, and polyacrylamide); polyethyleneimine; and cationic polymers.

Examples of the thickening agent include, for example: gum Arabic, carrageenan, gum karaya, gum tragacanth, carob gum, quince seed (marmelo), casein, dextrin, gelatin, sodium pectate, sodium araginate, methylcellulose, ethylcellulose, CMC, hydroxyethylcellulose, hydroxypropylcellulose, PVA, PVM, PVP, sodium polyacrylate, carboxyvinyl polymer, locust bean gum, guar gum, tamarind gum, cellulose dialkyldimethylammonium sulfate, xanthan gum, magnesium aluminum silicate, bentonite, hectorite, A1Mg silicate (veegum), laponite, anhydrous silicic acid, carbomer, and a (acrylates/alkyl acrylate (C10-30)) crosspolymer. The (acrylates/alkyl acrylate (C10-30)) crosspolymer functions as an active agent and an emulsifier.

Examples of the ultraviolet absorbers include, for example: benzoic acid-based ultraviolet absorbers (e.g., para-aminobenzoic acid (hereinafter abbreviated as PABA), PABA monoglyceryl esters, N,N-dipropoxy PABA ethyl esters, N,N-diethoxy PABA ethyl esters, N,N-dimethyl PABA ethyl esters, N,N-dimethyl PABA butyl esters, and N,N-dimethyl PABA ethyl esters); anthranilic acid-based ultraviolet absorbers (e.g., homomenthyl-N-acetyl anthranilate); salicylic acid-based ultraviolet absorbers (e.g., amyl salicylate, menthyl salicylate, homomenthyl salicylate, octyl salicylate, phenyl salicylate, benzyl salicylate, and p-isopropanol phenyl salicylate); cinnamic acid-based ultraviolet absorbers (e.g., octyl cinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, and glyceryl mono-2-ethylhexanoyl-diparamethoxycinnamate); benzophenone-based ultraviolet absorbers (e.g., 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone); 3-(4'-methylbenzylidene)-d,l-camphor and 3-benzylidene-d,l-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenylbenzotriazole; dibenzalazine; dianisoylmethane; 4-methoxy-4'-t-butyldibenzoylmethane; and 5-(3,3-dimethyl-2-norbornylidene)-3-pentan-2-one.

Examples of the humectants include, for example, glycerin, dynamite glycerin, dipropylene glycol, 1,3-butylene glycol, cholesteryl-12-hydroxystearate, sodium lactate, dl-pyrrolidone carboxylate, urea, and diglycerin ethylene oxide-propylene oxide adduct. In particular, when acid mucopolysaccharides, such as chondroitin sulfate, hyaluronic acid, or the like, are compounded, the whitening effect of alkoxysalicylic acid and/or its salts is improved.

Examples of the metal ion sequestrants include, for example, 1-hydroxyethane-1,1-diphosphonic acid, 1-hydroxyethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, and trisodium hydroxyethyl ethylenediamine triacetate.

Examples of the lower alcohols include, for example, ethanol, propanol, isopropanol, isobutyl alcohol, and t-butyl alcohol.

Examples of the polyhydric alcohols include, for example: dihydric alcohols (e.g., ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol); trihydric alcohols (e.g., glycerin and trimethylolpropane); tetrahydric alcohols (e.g., pentaerythritols, such as 1,2,6-hexanetriol and the like); pentahydric alcohols (e.g., xylitol and the like); hexahydric alcohols (e.g., sorbitol and mannitol); polyhydric alcohol polymers (e.g., diethylene glycol, dipropylene glycol, triethylene glycol, polypropylene glycol, tetraethylene glycol, diglycerin, polyethylene glycol, triglycerin, tetraglycerin, and polyglycerin); dihydric alcohol alkyl ethers (e.g., ethylene glycol monomethyl ether, ethylene glycol monoethyl ether, ethylene glycol monobutyl ether, ethylene glycol monophenyl ether, ethylene glycol monohexyl ether, ethylene glycol mono 2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether); dihydric alcohol alkyl ethers (e.g., diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether); dihydric alcohol ether esters (e.g., ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate); glycerin monoalkyl ethers (e.g., xyl alcohol, selachyl alcohol, and batyl alcohol); sugar alcohols (e.g., sorbitol, maltitol, maltotriose, mannitol, sucrose, erythritol, glucose, fructose, hydrolyzed starch, maltose, xylitol, and hydrogenated starch hydrolysate); glysolid; tetrahydrofurfuryl alcohol; POE-tetrahydrofurfuryl alcohol; POP-butyl ether; POP-POE-butyl ether; tripolyoxypropylene glycerol ether; POP-glycerol ether; POP-glycerol ether phosphate; and POP-POE-pentanerythritol ether, and polyglycerin).

Examples of monosaccharides include, for example: trioses (e.g., D-glyceryl aldehyde and dihydroxyacetone); tetroses (e.g., D-erythrose, D-erythrulose, D-threose, and erythritol); pentoses (e.g., L-arabinose, D-xylose, L-lyxose, D-arabinose, D-ribose, D-ribulose, D-xylulose, and L-xylulose); hexoses (e.g., D-glucose, D-talose, D-psicose, D-galactose, D-fructose, L-galactose, L-mannose, and D-tagatose); heptoses (e.g., aldoheptose and heplose); octoses (e.g., octulose); deoxy sugars (e.g., 2-deoxy-D-ribose, 6-deoxy-L-galactose, and 6-deoxy-L-mannose); amino sugars (e.g., D-glucosamine, D-galactosamine, sialic acid, aminouronic acid, and muramic acid); and uronic acids (e.g., D-glucuronic acid, D-mannuronic acid, L-guluronic acid, D-galacturonic acid, and L-iduronic acid).

Examples of oligosaccharides include, for example, sucrose, gentianose, umbelliferose, lactose, planteose, isolychnoses, α,α-trehalose, raffinose, lychnoses, umbilicin, and stachyoses and verbascoses.

Examples of polysaccharides include, for example, cellulose, quince seed, chondroitin sulfate, starch, galactan, dermatan sulfate, glycogen, gum Arabic, heparan sulfate, hyaluronic acid, tragacanth gum, keratan sulfate, chondroitin, xanthan gum, chondroitin sulfate, guar gum, dextran, keratan sulfate, locust bean gum, succinoglucan, and caloninic acid.

Examples of the amino acids include, for example: neutral amino acids (e.g., threonine and cysteine); and basic amino acids (e.g., hydroxylysine). Examples of amino acid derivatives include, for example, sodium acyl sarcosinate (sodium lauroyl sarcosinate), acyl glutamate, sodium acyl β-alaninate, glutathione, and pyrrolidone carboxylic acid.

Examples of the organic amines include, for example, monoethanolamine, diethanolamine, triethanolamine, morpholine, triisopropanolamine, 2-amino-2-methyl-1,3-propanediol, and 2-amino-2-methyl-1-propanol.

Examples of the polymer emulsions include acrylic resin emulsion, polyethyl acrylate emulsion, acrylic resin liquid, polyalkyl acrylate emulsion, polyvinyl acetate resin emulsion, and natural rubber latex.

Examples of the neutralizing agents include, for example: inorganic bases, such as sodium hydroxide, potassium hydroxide, and the like; and organic amines, such as monoethanolamine, diethanolamine, triethanolamine, morpholine, triisopropanolamine, 2-amino-2-methyl-1,3-propanediol, 2-amino-2-methyl-1-propanol, and the like.

Examples of the pH adjusters include, for example, buffers, such as lactic acid-sodium lactate, citric acid-sodium citrate, succinic acid-sodium succinate, and the like.

Examples of the vitamins include, for example, vitamins A, B1, B2, B6, C, and E, and their derivatives, pantothenic acid and its derivatives, and biotin.

Examples of the antioxidants include, for example, tocopherols, dibutylhydroxytoluene, butylhydroxyanisole, and gallic acid esters.

Examples of the antioxidant aids include, for example, phosphoric acid, citric acid, ascorbic acid, maleic acid, malonic acid, succinic acid, fumaric acid, cephalin, hexametaphosphate, phytic acid, and ethylenediaminetetraacetic acid.

Examples of the stabilizers include, for example, Na pyrosulfite and EDTA-2Na.

Examples of other ingredients that can be compounded include, for example, preservatives (e.g., phenoxyethanol, ethylparaben, butylparaben, and chlorphenesin); anti-inflammatory agents (e.g., glycyrrhizic acid derivatives, glycyrrhetinic acid derivatives, salicylic acid derivatives, hinokitiol, zinc oxide, and allantoin); whitening agents (e.g., placenta extract, saxifraga sarmentosa extract, arbutin, vitamin C, magnesium ascorbyl phosphate, ascorbyl glucoside, arbutin, and alkoxysalicylic acid and its salts); various extracts (e.g., phellodendron bark, coptis, lithospermum root, peony root, swertia herb, birch, sage, loquat leaf, ginseng, aloe, mallow, iris, grape, coix seed, loofah, lily, saffron, cnidium rhizome, ginger, hypericum erectum, ononis, garlic, capsicum, citrus peel, angelica root, and seaweed); activating agents (e.g., royal jelly, photosensitizers, and cholesterol derivatives); blood circulation promoters (e.g., nonanoyl vanillylamide, nicotinic acid benzyl ester, nicotinic acid β-butoxyethyl ester, capsaicin, zingerone, cantharides tincture, ichthammol, tannic acid, α-borneol, tocopherol nicotinate, inositol hexanicotinate, cyclandelate, cinnarizine, tolazoline, acetylcholine, verapamil, cepharanthine, and γ-oryzanol); anti-seborrheic agents (e.g., sulfur and thianthol); and anti-inflammatory agents (e.g., tranexamic acid, thiotaurine, and hypotaurine).

The oil-in-water emulsion composition according to the present invention is suitably used in various dosage forms, such as cream, emulsion, liquid, and the like. The product form may be skin care cosmetics, such as sunscreens and the like, or makeup cosmetics, such as makeup bases, foundations, and the like.

### EXAMPLES

Hereinafter, the present invention will be described in more detail by way of examples. However, the present invention is not limited thereto. Unless otherwise stated, amounts are all expressed as % by mass of the total amount.

### [Examples 1 to 4 and Comparative Examples 1 to 8]

Oil-in-water emulsion compositions of Examples 1 to 4 and Comparative Examples 1 to 8 were prepared by the following method in accordance with the compositions shown in Tables 1 to 3. Each of the oil-in-water emulsion compositions was evaluated for emulsion stability by the following method.

### [Preparation of oil-in-water emulsion composition]

(1) A corresponding thickener was added to a mixture of an aqueous phase prepared in accordance with each of the compositions shown in Tables 1 to 3, followed by mixing the resulting mixture uniformly.
(2) A corresponding inorganic powder was dispersed in a mixture of active agent and heat-melted oil prepared in accordance with each of the compositions shown in Tables 1 to 3.
(3) The dispersion prepared in (2) was added to the mixture prepared in (1), followed by emulsifying.

### [Emulsion stability]

A sample of each of the prepared oil-in-water emulsion compositions was evaluated for emulsion stability in accordance with the following evaluation criteria. Specifically, the prepared oil-in-water emulsion composition was charged into a tube, followed by being left at room temperature for one day, and its outer appearance on the following day was visually observed. When emulsion breakdown occurs, oil bleeding is observed.

In the following evaluation, "phase inversion" means a change from an oil-in-water (O/W) type to a water-in-oil (W/O) type.

### (Evaluation criteria)

A: The outer appearance is uniform, and no oil bleeding is observed.
B: Oil bleeding is observed
C: Phase inversion occurs during preparation of the oil-in-water emulsion composition.

The evaluation results of the emulsion stability of the oil-in-water emulsion compositions of Examples 1 to 4 and Comparative Examples 1 to 8 were as shown in Tables 1 to 3. The oil-in-water emulsion compositions evaluated as "A" in the above evaluation criteria were determined to be applicable as the present invention.

**[Table 1]**

| Oil-in-water emulsion composition | | Ex. 1 | Ex. 2 | Ex. 3 | Ex. 4 |
|---|---|---|---|---|---|
| Inorganic powder | Cetyl phosphate-treated silica-coated titanium oxide (Avg. primary particle diameter: 15 nm) | 4 | 4 | 4 | 4 |
| Active agent | PEG-60 hydrogenated castor oil | 1.5 | 1.5 | 1.5 | 3 |
| Thickener | (Dimethylacrylamide/Na acryloyldimethyl taurate) crosspolymer | 0.2 | | | 0.2 |
| | (Na acrylate/Na acryloyldimethyl taurate)copolymer (active content 37.5%) | | 0.5 | | |
| | (Ammonium acryloyldimethyl taurate/beheneth-25 methacrylate) crosspolymer | | | 0.2 | |
| Oil | Diisopropyl sebacate | 10 | 10 | 10 | 10 |
| | Ethylhexyl methoxycinnamate | 10 | 10 | 10 | 10 |
| | Polyglyceryl-6 polyricinoleate | 0.5 | 0.5 | 0.5 | 0.5 |
| | Dimethicone (6cs) | 3 | 3 | 3 | 3 |
| | Isododecane | 3 | 3 | 3 | 3 |
| Aqueous phase | (Acrylates/alkyl acrylate (C10-30)) crosspolymer | 0.08 | 0.08 | 0.08 | 0.08 |
| | Carbomer | 0.22 | 0.22 | 0.22 | 0.22 |
| | Xanthan gum | 0.1 | 0.1 | 0.1 | 0.1 |
| | Glycerin | 2 | 2 | 2 | 2 |
| | Ethanol | 7 | 7 | 7 | 7 |
| | Silica (particle diameter 2 µm to 10 µm) | 1.2 | 1.2 | 1.2 | 1.2 |
| | (Acrylamide/DMAPA acrylic acid/methoxy PEG methacrylic acid)copolymer (active content 10%) | 10 | 10 | 10 | |
| | K hydroxide | 0.3 | 0.3 | 0.3 | 0.3 |
| | Ion-exchanged water | Balance | Balance | Balance | Balance |
| Total | | 100 | 100 | 100 | 100 |
| Emulsion stability | | A | A | A | A |

**[Table 2]**

| Oil-in-water emulsion composition | | Comp. Ex. 1 | Comp. Ex. 2 | Comp. Ex. 3 | Comp. Ex. 4 |
|---|---|---|---|---|---|
| Inorganic powder | Cetyl phosphate-treated silica-coated titanium oxide (Avg. primary particle diameter: 15 nm) | 4 | 4 | | |
| | Al hydroxide and stearic acid-treated titanium oxide (Avg. primary particle diameter: 15 nm) | | | 4 | |
| | Al hydroxide and hydrogen dimethicone-treated titanium oxide (Avg. primary particle diameter: 15 nm) | | | | 4 |
| | Silica-coated titanium oxide (Avg. primary particle diameter: 15 nm) | | | | |
| Active agent | Cetyl phosphate | | | | |
| | PEG-60 hydrogenated castor oil | 1.5 | 1.5 | 1.5 | 1.5 |
| | PEG-100 stearate | | | | |
| | PEG-60 glyceryl isostearate | | | | |
| | Beheneth-20 | | | | |
| Thickener | (Dimethylacrylamide/Na acryloyldimethyl taurate) crosspolymer | | | 0.2 | 0.2 |
| | Stearoxyhydroxypropyl methylcellulose | | 0.06 | | |
| Oil | Diisopropyl sebacate | 10 | 10 | 10 | 10 |
| | Ethylhexyl methoxycinnamate | 10 | 10 | 10 | 10 |
| | Polyglyceryl-6 polyricinoleate | 0.5 | 0.5 | 0.5 | 0.5 |
| | Dimethicone (6cs) | 3 | 3 | 3 | 3 |
| | Isododecane | 3 | 3 | 3 | 3 |
| Aqueous phase | (Acrylates/alkyl acrylate (C10-30)) crosspolymer | 0.08 | 0.08 | 0.08 | 0.08 |
| | Carbomer | 0.22 | 0.22 | 0.22 | 0.22 |
| | Xanthan gum | 0.1 | 0.1 | 0.1 | 0.1 |
| | Glycerin | 2 | 2 | 2 | 2 |
| | Ethanol | 7 | 7 | 7 | 7 |
| | Silica (particle diameter 2 µm to 10 µm) | 1.2 | 1.2 | 1.2 | 1.2 |
| | (Acrylamide/DMAPA acrylic acid/methoxy PEG methacrylic acid)copolvmer (active content 10%) | 10 | 10 | 10 | 10 |
| | K hydroxide | 0.3 | 0.3 | 0.3 | 0.3 |
| | Water | Balance | Balance | Balance | Balance |
| Total | | 100 | 100 | 100 | 100 |
| Emulsion stability | | B | B | B | C |

**[Table 3]**

| Oil-in-water emulsion composition | | Comp. Ex. 5 | Comp. Ex. 6 | Comp. Ex. 7 | Comp. Ex. 8 |
|---|---|---|---|---|---|
| Inorganic powder | Cetyl phosphate-treated silica-coated titanium oxide (Avg. primary particle diameter: 15 nm) | | 4 | 4 | 4 |
| | Al hydroxide and stearic acid-treated titanium oxide (Avg. primary particle diameter: 15 nm) | | | | |
| | Al hydroxide and hydrogen dimethicone-treated titanium oxide (Avg. primary particle diameter: 15 nm) | | | | |
| | Silica-coated titanium oxide (Avg. primary particle diameter: 15 nm) | 4 | | | |
| Active agent | Cetyl phosphate | 0.4 | | | |
| | PEG-60 hydrogenated castor oil | 1.5 | | | |
| | PEG-100 stearate | | 1.5 | | |
| | PEG-60 glyceryl isostearate | | | 1.5 | |
| | Beheneth-20 | | | | 1.5 |
| Thickener | (Dimethylacrylamide/Na acryloyldimethyl taurate) crosspolymer | 0.2 | 0.2 | 0.2 | 0.2 |
| | Stearoxyhydroxypropyl methylcellulose | | | | |
| Oil | Diisopropyl sebacate | 10 | 10 | 10 | 10 |
| | Ethylhexyl methoxycinnamate | 10 | 10 | 10 | 10 |
| | Polyglyceryl-6 polyricinoleate | 0.5 | 0.5 | 0.5 | 0.5 |
| | Dimethicone (6cs) | 3 | 3 | 3 | 3 |
| | Isododecane | 3 | 3 | 3 | 3 |
| Aqueous phase | (Acrylates/alkyl acrylate (C10-30)) crosspolymer | 0.08 | 0.08 | 0.08 | 0.08 |
| | Carbomer | 0.22 | 0.22 | 0.22 | 0.22 |
| | Xanthan gum | 0.1 | 0.1 | 0.1 | 0.1 |
| | Glycerin | 2 | 2 | 2 | 2 |
| | Ethanol | 7 | 7 | 7 | 7 |
| | Silica (particle diameter 2 µm to 10 µm) | 1.2 | 1.2 | 1.2 | 1.2 |
| | (Acrylamide/DMAPA acrylic acid/methoxy PEG methacrylic acid)copolymer (active content 10%) | 10 | 10 | 10 | 10 |
| | K hydroxide | 0.3 | 0.3 | 0.3 | 0.3 |
| | Water | Balance | Balance | Balance | Balance |
| Total | | 100 | 100 | 100 | 100 |
| Emulsion stability | | C | C | C | B |

The oil-in-water emulsion compositions of Examples 1 to 4, including all the components (A) to (C), were good in terms of the emulsion stability.

In Comparative Examples 1 and 2, not including the acrylamide-based thickener, i.e., component (C), the emulsification became unstable, and oil bleeding was observed.

The inorganic powder included in Comparative Examples 3 to 5 was not the inorganic powder treated with the monoalkyl phosphate, i.e., component (A). Thus, the inorganic powder could not exist stably in the oil, and oil bleeding was observed in Comparative Example 3. In Comparative Examples 4 and 5, oil-in-water emulsion compositions could not be prepared due to phase inversion.

The polyoxyethylene hydrogenated castor oil, i.e., component (B) was not included in Comparative Examples 6 to 8. Thus, the emulsification became unstable. As a result, oil-in-water emulsion compositions could not be prepared due to phase inversion in Comparative Examples 6 and 7, and oil bleeding was observed in Comparative Example 8.

The treatment agents used for the inorganic powders, i.e., "cetyl phosphate-treated silica-coated titanium oxide", "Al hydroxide and stearic acid-treated titanium oxide", and "Al hydroxide and hydrogen dimethicone-treated titanium oxide" all have high safety for the human body. Further, "cetyl phosphate-treated silica-coated titanium oxide" and "Al hydroxide and stearic acid-treated titanium oxide" are not microplastics, and have a low load on the natural environment.

However, since "Al hydroxide and stearic acid-treated titanium oxide" and "Al hydroxide and hydrogen dimethicone-treated titanium oxide" are not the inorganic powders treated with the monoalkyl phosphate, these could not stably exist in oil. Thus, the oil-in-water emulsion compositions of Comparative Examples 3 and 4 using these were poor in terms of the emulsion stability.

As described above, the oil-in-water emulsion compositions satisfying the configuration of the present invention are found to be oil-in-water emulsion compositions having high safety and good emulsion stability.

Further, according to the present invention, it is possible to provide the oil-in-water emulsion composition having a low load on the natural environment.

Embodiments of the present invention include, for example, the following.
<1> An oil-in-water emulsion composition, including:
   (A) an inorganic powder treated with a monoalkyl phosphate;
   (B) a polyoxyethylene hydrogenated castor oil; and
   (C) an acrylamide-based thickener.
<2> The oil-in-water emulsion composition according to <1>, wherein the monoalkyl phosphate includes cetyl phosphate.
<3> The oil-in-water emulsion composition according to <1> or <2>, wherein the inorganic powder is an inorganic powder including a silica coating.
<4> The oil-in-water emulsion composition according to any one of <1> to <3>, wherein the inorganic powder is a titanium oxide powder.
<5> The oil-in-water emulsion composition according to any one of <1> to <4>, wherein an average number of moles of ethylene oxide added in (B) the polyoxyethylene hydrogenated castor oil is 50 mol or more and 100 mol or less.

The oil-in-water emulsion composition according to any one of <1> to <5> can solve various conventional problems and achieve the object of the present invention.

The present application claims priority to Japanese Patent Application No. 2023-223525 filed with the Japan Patent Office on December 28, 2023, and the entire contents of this application are incorporated herein by reference.

## Claims

1. An oil-in-water emulsion composition, comprising:
(A) an inorganic powder treated with a monoalkyl phosphate;
(B) a polyoxyethylene hydrogenated castor oil; and
(C) an acrylamide-based thickener.

2. The oil-in-water emulsion composition according to claim 1, wherein
the monoalkyl phosphate includes cetyl phosphate.

3. The oil-in-water emulsion composition according to claim 1 or 2, wherein
the inorganic powder is an inorganic powder including a silica coating.

4. The oil-in-water emulsion composition according to claim 1 or 2, wherein
the inorganic powder is a titanium oxide powder.

5. The oil-in-water emulsion composition according to claim 1 or 2, wherein
an average number of moles of ethylene oxide added in (B) the polyoxyethylene hydrogenated castor oil is 50 mol or more and 100 mol or less.
